# EUROPÄISCHE PATENTANMELDUNG

(11) **EP 1 759 636 A2**
(43) Veröffentlichungstag der Anmeldung: **07.03.2007**
(21) Anmeldenummer: 06016908.3
(22) Anmeldetag: 14.08.2006
(51) Int. Cl.: A61B 6/00, A61B 6/14

(54) **Vorrichtung zur Erstellung von Durchstrahlungsbildern**

(30) Priorität: 25.08.2005 DE 102005040375
(71) Anmelder: Dürr Dental GmbH & Co. KG, 74321 Bietigheim-Bissingen (DE)
(72) Erfinder: Thoms, Michael, 74321 Bietigheim-Bissingen (DE)
(74) Vertreter: Ostertag, Reinhard

(57) **Zusammenfassung**

Eine Vorrichtung zur Erstellung von Durchstrahlungsbildern umfasst eine Strahlungsquelle (12), eine Bild-Detektionseinheit (32), die mit der Strahlung beaufschlagt ist, welche mit einem zu untersuchenden Objekt (30) wechselgewirkt hat, sowie eine Dokumentationseinrichtung (88). Wenigstens ein Betriebsparameter der Vorrichtung ist einstellbar und von der Dokumentationseinrichtung (88) registrierbar. Es ist wenigstens ein Signalgenerator ((80, 29, 63, 35) vorgesehen, der ein mindestens einem einstellbaren Betriebsparameter entsprechendes Signal erzeugt. Der Signalgenerator (80, 29, 63, 35) ist über eine Datenübertragungsstrecke (84; 100, 76, 60, 50, 94; 106; 112) mit einem Dateneingang (82) der Dokumentationseinrichtung (88) verbunden.

## Beschreibung

Die Erfindung betrifft eine Vorrichtung zur Erstellung von Durchstrahlungsbildern mit
a) einer Strahlungsquelle;
b) einer Bild-Detektionseinheit, die mit der Strahlung beaufschlagt ist, welche mit einem zu untersuchenden Objekt wechselgewirkt hat; und
c) einer Dokumentationseinrichtung;
   wobei
d) wenigstens ein Betriebsparameter der Vorrichtung einstellbar und von der Dokumentationseinrichtung registrierbar ist.

Derzeit wird als Bildwandler bei der Röntgendiagnostik entweder der klassische Röntgenfilm oder ein digitaler Bildwandler in Form einer Speicherfolie oder eines CCD-Sensors verwendet.

Aufgrund gesetzlicher Vorschriften muß die während der Aufnahme abgegebene Röntgendosis dokumentiert werden, z. B. in einer Datenbank abgespeichert werden. Die Röntgendosis wird dabei in Form der Parameter Röhrenspannung, Strahlstrom und Belichtungszeit erfasst.

Diese Parameter werden in der Regel per Hand nach Erfahrenswerten eingestellt. Sie könnten aber auch vor der Röntgenaufnahme durch ein Rechnerprogramm anhand von gespeicherten Daten vorgeschlagen werden.

Bei der dentalen Röntgendiagnostik beispielsweise umfasst eine entsprechende Datenbank Betriebsparameter für die Aufnahme unterschiedlicher Kieferregionen, Angaben zu den Details, auf die es besonders ankommt, Angaben zum Zweck der Untersuchung, (z. B. Entzündungsherde bestimmen, Remineralisierungskontrolle, Werkzeugpositionierung in einem Wurzelkanal usw.) oder sonstige zu berücksichtigende Aufnahmespezifikationen.

Die festzuhaltenden Betriebsparameter werden in Praxen mit EDV-Ausstattung typischerweise mittels eines Dokumentationsrechners in eine Patienten-Datenbank eingegeben.

Die jeweilige manuelle Eingabe entweder der Betriebsparameter an der Röntgenröhre oder der Betriebsparameter in den Dokumentationsrechner ist jedoch recht mühsam und zeitaufwendig.

Die Erfindung hat die Aufgabe, die Dokumentation der Röntgendosis in Verbindung mit der Röntgenaufnahme zu vereinfachen.

Dies wird ausgehend vom eingangs geschilderten Stand der Technik dadurch gewährleistet, daß
e) wenigstens ein Signalgenerator vorgesehen ist, der ein mindestens einem einstellbaren Betriebsparameter entsprechendes Signal erzeugt; und
f) der mindestens eine Signalgenerator über eine Datenübertragungsstrecke mit einem Dateneingang der Dokumentationseinrichtung verbunden ist.

Das von dem Signalgenerator erzeugte Signal liefert automatisch die Information über einen oder mehrere zu dokumentierende Betriebsparameter. Diese Betriebsparameter werden ohne menschliches Eingreifen auf die Dokumentationseinrichtung, beispielsweise einen Dokumentationsrechner, übertragen und müssen somit nicht mehr von Hand eingegeben werden.

Vorteilhafte Ausführungen der Erfindung sind in den abhängigen Ansprüchen angegeben:

Der Signalgenerator ist gemäß Anspruch 2 vorteilhafterweise der Strahlungsquelle zugeordnet. Damit können die Betriebsparameter automatisch erfasst werden, die die Röntgendosis bestimmen.

Es ist vorteilhaft, wenn gemäß Anspruch 3 ein steuerbares Netzteil und/oder wenigstens ein steuerbarer Aktuator und/oder wenigstens eine weitere steuerbare Komponente vorgesehen sind, deren Dateneingänge Daten von der Dokumentationseinrichtung erhalten. Damit ist die Vorrichtung an die spezifischen Aufnahmebedingungen anpassbar, wobei die steuerbaren Bauteile nicht manuell, sondern mittels der Dokumentionseinrichtung angesteuert werden können.

Die Maßnahme nach Anspruch 4, wonach die oben erwähnten zusätzlichen steuerbaren Bauteile ebenfalls einen Signalgenerator aufweisen, ermöglicht die Erfassung und Überwachung einer Vielzahl den Betrieb der Vorrichtung bestimmender Betriebsparameter.

Ist die Datenübertragungsstrecke nach Anspruch 5 in den dort beschriebenen wenigstens zwei Modi betreibbar, ist in vorteilhafter Weise ein Datenaustausch in beiden Richtungen zwischen den miteinander verbundenen Komponenten möglich.

Stellt eine der angesteuerten Komponenten eine Kopie der von ihr erhaltenen Daten wieder auf der Datenübertragungsstrecke bereit, wie es in Anspruch 6 angegeben ist, so kann diese Kopie verwendet werden, um die fehlerfreie Übertragung der entsprechenden Daten zu überwachen, was wiederum die Betriebssicherheit erhöht.

Ein solcher Datenaustausch ist auf einfache Weise zu gewährleisten, wenn an wenigstens einem Ende einer Datenübertragungsstrecke gemäß Anspruch 7 eine digitale Schnittstelle vorgesehen ist.

Die Betriebssicherheit der Vorrichtung wird weiter erhöht, wenn gemäß Anspruch 8 die Datenübertragungsstrecke eine galvanische Trennung gewährleistet.

Eine galvanische Trennung ist nach Anspruch 9 auf vorteilhafte Weise realisierbar, indem die Datenübertragungsstrecke zumindest in einem Teil eine optische Übertragungsstrecke ist.

Dies wiederum ist einfach umzusetzen, indem die Datenübertragungsstrecke nach Anspruch 10 optoelektronische Bauelemente umfaßt.

Eine andere sichere und vorteilhafte Ausbildung einer galvanisch getrennten Datenübertragungsstrecke ist es gemäß Anspruch 11, zumindest teilweise eine Funkstrecke vorzusehen.

Vorteilhaft ist außerdem, wenn der oder die einstellbaren Betriebsparameter nach Anspruch 12 die Röhrenspannung und/oder die Belichtungszeit und/oder den Strahlstrom und/oder die Gerätenummer und/oder die Wellenlänge der Strahlung und/oder den Strahlquerschnitt und/oder die Lage des Patienten oder dergleichen umfaßt.

Die Erstellung von Durchstrahlungsbildern gelingt auf einfache Weise, wenn die Strahlungsquelle nach Anspruch 13 Röntgenstrahlung bereitstellt.

Vorteilhaft wird bei der Anwendung von Röntgenstrahlung als Bildwandler gemäß Anspruch 14 ein CCD-Sensor eingesetzt, da ein solcher ein digitales Bild bereitstellt, das leicht archivierbar und bearbeitbar ist, und der eine solch hohe Empfindlichkeit aufweist, so daß mit kleiner Dosis gearbeitet werden kann.

Ähnliche Vorteile erhält man, wenn man gemäß Anspruch 15 als Bildwandler eine Speicherfolie vorsieht.

Die Betriebssicherheit wird weiter erhöht, wenn das steuerbare Netzteil gemäß Anspruch 16 bei einer Abweichung zwischen wenigstens einem Soll- und Ist-Betriebsparameter von der Dokumentationseinrichtung und/oder der Bildverarbeitungseinrichtung ein Signal erhält, welches den Betrieb der Strahlungsquelle unterbindet.

Ferner ist es vorteilhaft, wenn die räumliche Lage des durchstrahlten Objekts gemäß Anspruch 17 durch den wenigstens einen steuerbaren Aktuator einstellbar ist.

Nachfolgend wird eine erfindungsgemäße Vorrichtung unter Bezugnahme auf die Zeichnung näher erläutert. Darin zeigen:
- Figur 1: ein Blockschaltbild einer Röntgenvorrichtung mit integrierter Dokumentation und Sollwertvorgabe;
- Figur 2: eine ähnliche Darstellung wie Figur 1, in welcher ein zweites Ausführungsbeispiel gezeigt ist;
- Figur 3: eine optische Datenübertragungsstrecke, die bei einer Röntgenvorrichtung nach Figur 1 oder 2 Verwendung finden kann; und
- Figur 4: eine Funk-Datenübertragungsstrecke, die bei einer Röntgenvorrichtung nach Figur 1 oder 2 Verwendung finden kann.

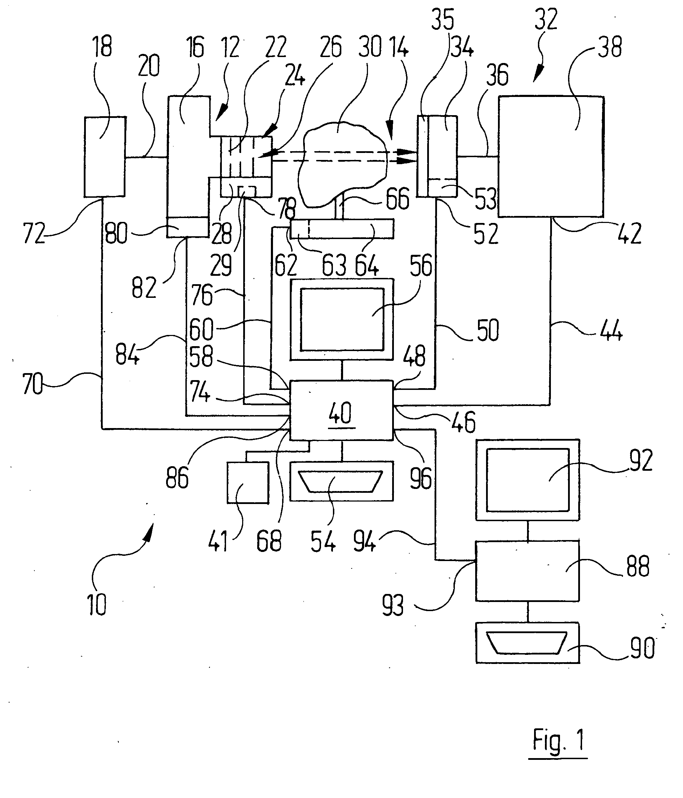

Figur 1 zeigt ein erstes Ausführungsbeispiel einer Vorrichtung 10 zur Erstellung von Durchstrahlungsbildern mit einer Strahlungsquelle 12, welche Röntgenstrahlung erzeugt. Die Röntgenstrahlung ist durch gestrichelte Pfeile 14 angedeutet.

Die Strahlungsquelle 12 umfaßt eine Röntgenröhre 16 üblicher Bauart, welche durch ein steuerbares Hochspannungs-Netzteil 18 über eine Versorgungsleitung 20 mit Energie versorgt wird. Die Betriebsparameter der Röntgenröhre (Röhrenspannung, Strahlstrom, Belichtungszeit) werden durch Steuersignale vorgegeben, die auf Steuereingänge des Netzteiles 18 gegeben werden, wie noch genauer beschrieben wird.

Die innerhalb der Röntgenröhre 16 erzeugte Röntgenstrahlung 14 verläßt die Röntgenröhre 16 durch ein für Röntgenstrahlung durchlässiges Austrittsfenster 22. Von dort aus durchläuft die Röntgenstrahlung 14 ein Blendensystem 24 mit Blenden 26, mittels derer der Strahlquerschnitt des Röntgenstrahls einstellbar ist. Die Positionen der Blenden 26 werden über eine steuerbare Blenden-Antriebseinheit 28 eingestellt, welche eine Mehrzahl unterschiedlicher Verstellrichtungen zugeordneter Aktuatoren umfassen kann.

Nachdem die Röntgenstrahlung 14 ein Objekt 30, beispielsweise eines Kieferabschnitts eines Patienten, durchdrungen und also mit diesem wechselgewirkt hat, trifft sie auf eine Bild-Detektionseinheit 32. Diese kann als eigentliches Aufnahmemedium beispielsweise einen herkömmlichen Röntgenfilm oder eine ein elektronisches Bild bereitstellende Aufnahmeeinheit aufweisen.

Bei dem Ausführungsbeispiel nach Figur 1 umfaßt eine digitale Detektionseinheit 32 einen CCD-Sensors 34 mit einem vorgeschalteten Lumineszenzschild 35. Der Ausgang des CCD-Sensors 34 steht über eine Leitung 36 mit einer Ausleseeinheit 38 in Verbindung. Letztere stellt die für den Betrieb des CCD-Sensors 34 notwendigen Spannungen und Steuersignale bereit und liest das im CCD-Sensor 34 gespeicherte Bild aus und überstellt dieses einem Rechner 40, der zur Bildverarbeitung und zu Steuerungszwecken vorgesehen ist.

Eine andere digitale Detektionseinheit 32 kann eine röntgenempfindliche Speicherfolie 34 umfassen. Die Ausleseeinheit 38 ist dann eine Speicherfolien-Ausleseeinheit 38 (Scanner), wie sie üblicherweise bei Speicherfolien verwendet wird. Die elektrische Leitung 36 ist dann durch die optische Auslesestrecke des Scanners ersetzt.

Eine Schnittstelle 42 der Ausleseeinheit 38 des hier eingesetzten CCD-Sensors 34 ist über eine Datenübertragungsleitung 44 mit einer Schnittstelle 46 des Rechners 40 verbunden. Eine Schnittstelle 48 des Rechners 40 wiederum ist über eine Datenübertragungsleitung 50 mit einer Schnittstelle 52 des CCD-Sensors 34 verbunden, über die Arbeitsparameter des CCD-Sensor 34 gesteuert werden können. Der CCD-Sensor 34 weist zudem einen Signalgenerator 53 (z. B. einen Speicherbereich) auf, welcher ein den Arbeitseinstellungen des CCD-Sensors 34 entsprechendes Signal erzeugt und der ebenfalls über die Schnittstelle 52 mit dem Rechner 40 kommuniziert.

Der Rechner 40 umfaßt ein Tastenfeld 54, über welches Befehle eingegeben werden können, sowie einen Monitor 56, auf welchem z.B. eingegebene Befehle oder aktuelle Betriebsparameter angezeigt werden. Beispiele für eingegebene Befehle sind z. B. Betriebsspannung, Strahlstrom und Belichtungszeit der Röntgenröhre, Arbeitsspannung und Auslesefrequenz des CCD-Sensors, usw.. Insofern dient der Rechner 40 zusätzlich als Bedien- bzw. Steuerrechner.

Eine Datenübertragungsleitung 60 verbindet eine Schnittstelle 58 des Rechners 40 mit einer Schnittstelle 62 einer Objekt-Positioniereinheit 64, die einen Signalgenerator 63 aufweist. Mit Hilfe der Objekt-Positioniereinheit 64 ist die räumliche Lage des Objekts 30 in Bezug auf die Röntgenquelle 12 und den CCD-Sensor 34 in x-, y- und z-Richtung einstellbar. Die Positioniereinheit 64 steuert dazu ein mit dem Objekt 30 lösbar verbundenes bewegliches Positioniermittel 66, im Fall einer Kiefer-Röntgenaufnahme beispielsweise eine Gebiß-Auflage, mittels (nicht dargestellter) Elektromotoren an.

Eine weitere Schnittstelle 68 des Rechners 40 ist über eine Datenübertragungsleitung 70 mit einer Schnittstelle 72 des steuerbaren Netzteiles 18 verbunden.

Außerdem kommuniziert der Rechner 40 mittels einer Schnittstelle 74 über eine Datenübertragungsleitung 76 mit einer Schnittstelle 78 der Blenden-Antriebseinheit 28. Diese weist einen Signalgenerator 29 auf, der über die Schnittstelle 78 kommunizieren kann und ein der Stellung der Blenden 26 entsprechendes Signal bereitstellt.

Die Strahlungsquelle 12 umfaßt einen Signalgenerator 80, welcher den die resultierende Röntgenstrahlung 14 bestimmenden Betriebsparametern, d.h. Röhrenspannung, Strahlstrom und Belichtungszeit, entsprechende Signale erzeugt. Eine Schnittstelle 82 des Signalgenerators 80 ist über eine Datenübertragungsleitung 84 mit einer Schnittstelle 86 des Rechners 40 verbunden.

Eine Dokumentationseinrichtung in Form eines Dokumentationsrechners 88 mit Tastenfeld 90 und Monitor 92 hat eine Schnittstelle 93, die über eine Leitung 94 mit einer weiteren Schnittstelle 96 des Rechners 40 verbunden ist. Sie erhält von dort die verschiedenen Betriebsparameter der Röntgenvorrichtung.

Der Dokumentationsrechner 88 dient dazu, die gemäß Röntgenverordnung anzugebenden Betriebsparameter Röhrenspannung, Strahlstrom und Belichtungszeit in Verbindung mit einer Kennung für die getätigte Aufnahme, zum Beispiel einer der Aufnahme zugeordneten Aufnahmenummer, und den zugehörigen Patientendaten zu speichern, so daß die Röntgendosis, der ein Patient im Laufe der Zeit ausgesetzt ist, dauerhaft dokumentiert wird.

Die oben beschriebenen, mit dem Rechner 40 verbundenen gesteuerten Komponenten sind in bekannter Weise derart konstruiert, daß die über eine entsprechende Schnittstelle empfangenen Steuerungs-Signale elektronisch und/oder mechanisch umgesetzt werden und die Komponenten entsprechend diesen Signale eingestellt werden.

Auf dem Rechner 40 ist eine entsprechende Software installiert, welche die Kommunikation mit den an den Rechner 40 angeschlossenen steuerbaren Komponenten koordiniert. Somit sind verschiedene Betriebsparameter über den Rechner 40 einstellbar:

Das steuerbare Netzteil 18 empfängt über die Datenübertragungsleitung 70 und seine Schnittstelle 72 Parameter für die Röhrenspannung, den Strahlungsstrom und die Belichtungszeit.

Die Schnittstelle 78 der Blenden-Antriebseinheit 28 empfängt ein Signal, anhand dessen die Stellung der Blenden justiert wird, so daß ein bestimmter Strahlquerschnitt erhalten wird.

Aufgrund der in den Rechner 40 eingegebenen Informationen stellt die Objekt-Positioniereinheit 64 die Lage des Objektes ein, nachdem sie über ihre Schnittstelle 62 und die Datenübertragungsleitung 60 entsprechende PositionsSignale empfangen hat.

Das Arbeiten und Auslesen der Bild-Detektionseinheit 32 steuernde Signale werden über die Schnittestelle 42 auf die Ausleseeinheit 38 gegeben.

Nachdem die zur Aufnahme benötigten Betriebsparameter in den Rechner 40 eingegeben, von dort zu den verschiedenen Komponenten übertragen und diese entsprechend eingestellt wurden, erfolgt die eigentliche Bildaufnahme: Erzeugung des Röntgenlichtes und Generieren des dem durch das Objekt 30 durchgelassenen Anteils des Röntgenlichtes entsprechenden Strahlungsbildes.

Die Ausleseeinheit 38 liest dann Bildsignale aus dem CCD-Sensor 34 aus, welche über die Datenübertragungsleitung 44 an die Schnittstelle 46 des Rechners 40 übertragen werden und dort von einer entsprechenden Software in ein sichtbares Bild umgewandelt werden, welches auf dem Monitor 56 dargestellt wird und zusätzlich auf einem Drucker 41 ausgegeben wird.

Der Aufnahme wird ferner vom Dokumentationsrechner 88 zu Dokumentationszwecken eine fortlaufende Aufnahmenummer zugeordnet.

Aus den Signalen des Signal-Generators 80 kennt der Dokumentationsrechner 88 die Betriebsparameter der Röntgenquelle 12, und dieser speichert nun die Betriebsparameter zusammen mit der Aufnahme-Nummer und den zugehörigen Patientendaten.

Somit entfällt ein manuelles Eingeben der Betriebsparameter der Röntgenquelle 12 in den Dokumentationsrechner 88 über das Tastenfeld 90.

Zusätzlich zu den die Strahlendosis vorgebenden Betriebsparametern werden auch die anderen einstellbaren Betriebsparameter (Stellungen der Blenden-Antriebseinheit 28, der der Objekt-Positioniereinheit 64, Betriebsparamer der Bild-Detektionseinheit 32), die vom Rechner 40 über die Leitung 94 bereitgestellt werden, vom Dokumentationsrechner 88 abgespeichert.

Die zu einer bestimmten Aufnahme gehörenden Betriebsparameter sind auch umgekehrt von dem Dokumentationsrechner 88 auf den Rechner 40 übertragbar, wodurch für spätere Aufnahmen die Eingabe der einzelnen Betriebsparameter ganz oder teilweise entfallen kann und die Dauer des Einstellvorgangs deutlich verkürzt wird.

Die Schnittstellen 72, 78, 62 und 52 der steuerbaren Komponenten (Netzteil 18, Blenden-Antriebseinheit 28, Objekt-Positionseinheit 64 bzw. Bildwandler 32) empfangen somit Steuerdaten, welche von dem Rechner 88 übertragen werden. Der Rechner 40 dient somit auch als Datenweiche und koordiniert den Datenfluß.

Dementsprechend können dem Datenbestand des Dokumentationsrechners 88 Soll-Betriebsparameter für neue Aufnahmen entnommen werden. Die einzelnen steuerbaren Komponenten sind derart ausgebildet, daß sie ihre jeweiligen Ist-Betriebsparameter an den Rechner 40 zurückmelden. Die Datenübertragungsstrecken zwischen Rechner 40 und steuerbaren Komponenten sind also in zwei Modi betreibbar, wobei im ersten Modus die Ist-Betriebsparameter der Strahlungsquelle 12 und der anderen gesteuerten Komponenten zum Rechner 40 übertragen werden.

Im zweiten Modus werden Soll-Betriebsparameter von dem Dokumentationsrechner 88 über den Rechner 40 an die Strahlungsquelle 12 und die anderen steuerbaren Komponenten übertragen.

Der jeweilige Modus wird dabei vom Rechner 40 softwaregesteuert ausgewählt. Ein kontinuierlicher Datenaustausch der Ist- und Soll-Betriebsparameter ermöglicht eine kontinuierliche Überwachung des korrekten Arbeitens der Röntgenvorrichtung.

Dabei kann jede steuerbare Komponente die empfangenen Daten, die als Grundlage ihrer Einstellung dienen, in Kopie zurücksenden. Dieses bestätigt zum einen den Empfang der Daten, kann zum anderen aber auch dafür verwendet werden, die empfangenen Daten mit den von dem Rechner 40 bzw. dem Dokumentationsrechner 88 gesendeten Daten zu vergleichen.

Weichen die zurückgesendeten Daten von den gesendeten Daten ab, so unterbindet der Rechner 40 die Aktivierung des steuerbaren Netzteils 18, so daß keine Energieversorgung der Röntgenröhre 16 erfolgt.

Gleichzeitig erscheint ein entsprechender Warnhinweis auf dem Monitor 56 des Rechners 40, so daß das Bedienpersonal die Information erhält, welcher oder welche Betriebsparameter den Soll-Vorgaben nicht entspricht.

Dies verhindert einerseits fehlerhafte Aufnahmen, die im schlechtesten Fall eine erneute Aufnahme und damit eine erneute Strahlenbelastung eines Patienten erforderlich machen. Zum anderen wird eine schnelle Fehlerdiagnose ermöglicht, da der fehlerhafte Betriebsparameter bzw. die falsch eingestellte Vorrichtungs-Komponente direkt angezeigt wird.

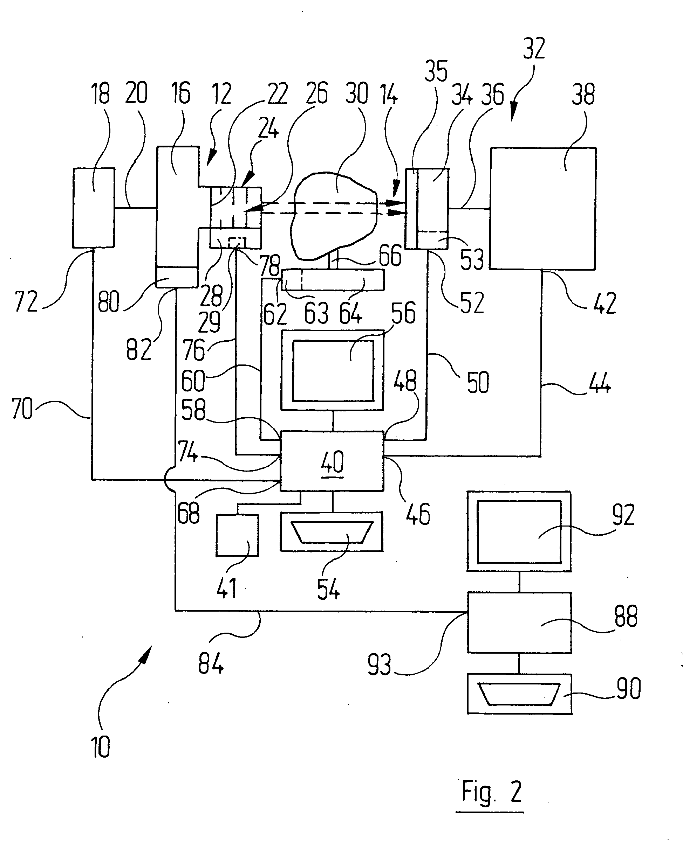

Figur 2 zeigt ein zweites Ausführungsbeispiel einer Vorrichtung zur Erstellung von Durchstrahlungsbildern. Darin sind der Figur 1 entsprechende Komponenten mit den selben Bezugszeichen gekennzeichnet.

Der Unterschied zu dem Ausführungsbeispiel nach Figur 1 besteht einzig darin, daß der Dokumentationsrechner 88 nun direkt mit dem Signalgenerator 80 der Strahlungsquelle 12 in Verbindung steht.

Ein Betreiben der Vorrichtung in den oben beschriebenen zwei Betriebsmodi ist auch bei dem Ausführungsbeispiel nach Figur 2 möglich. Dort steht der Signalgenerator 80 der Strahlungsquelle 12 mit dem Dokumentationsrechner 88 in Kommunikationsverbindung. Dementsprechend stehen als überprüfbare Betriebsparameter diejenigen zur Verfügung, welche durch das steuerbare Netzteil 18 eingestellt werden.

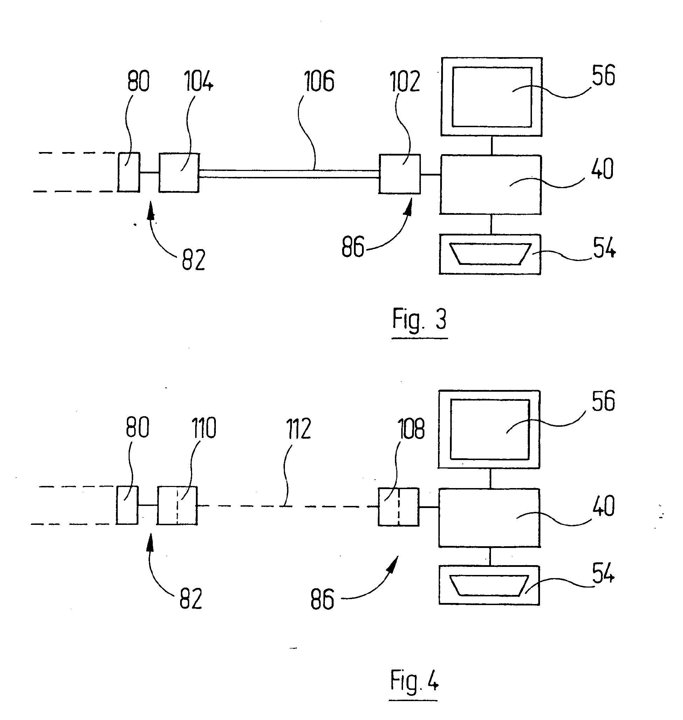

Die Figuren 3 und 4 zeigen zwei Ausführungsbeispiele einer Datenübertragungsstrecke, welche eine galvanische Trennung zwischen den miteinander kommunizierenden Komponenten gewährleisten.

Dabei tragen den Figuren 1 und 2 funktionell entsprechende Komponenten die selben Bezugszeichen.

Figur 3 zeigt die Datenübertragungsstrecke zwischen dem Rechner 40 und dem Signalgenerator 80 der Strahlungsquelle 12. Zur galvanischen Trennung weisen sowohl die Schnittstelle 86 des Rechners 40 als auch die Schnittstelle 82 des Signalgenerators 80 Optokopplereinheiten 102 bzw. 104 auf, die über einen Lichtleiter 106 miteinander in Verbindung stehen. Die Datenübertragung erfolgt also auf optischem Weg.

Diese Art der Datenübertragung kann auch bei allen anderen in den Figuren 1 und 2 gezeigten Datenübertragungsstrecken eingesetzt werden, wodurch sich eine vollständige galvanische Trennung der Rechner 40 und/oder 88 von den mit ihnen verbundenen Komponenten 18, 80, 28, 64 und 32 ergibt.

Figur 4 zeigt ein weiteres Ausführungsbeispiel einer galvanischen getrennten Datenübertragungstrecke. Darin weisen die Schnittstellen 86 des Rechners 40 und 82 des Signalgenerators 80 jeweils Funkmodemteile 108 bzw. 110 auf, die zum Senden und zum Empfang von Funksignalen geeignet sind. Eine Datenübertragungsstrecke 112 wird somit vollständig leitungslos überbrückt.

Auch diese Art der Datenübertragung kann für alle in den Figuren 1 und 2 gezeigten Datenübertragungsstrecken eingesetzt werden.

Durch den Einsatz von optischer Datenübertragung und/oder einer Datenübertragung via Funksignal werden Störfaktoren vermindert. Zudem können Geräte, die unterschiedlichen Normen entsprechen, in einfacher Weise miteinander kombiniert werden.

## Patentansprüche

1. Vorrichtung zur Erstellung von Durchstrahlungsbildern mit
a) einer Strahlungsquelle (12);
b) einer Bild-Detektionseinheit (32), die mit der Strahlung beaufschlagt ist, welche mit einem zu untersuchenden Objekt (30) wechselgewirkt hat; und
c) einer Dokumentationseinrichtung (88);
wobei
d) wenigstens ein Betriebsparameter der Vorrichtung einstellbar und von der Dokumentationseinrichtung (88) registrierbar ist,
**dadurch gekennzeichnet, daß**
e) wenigstens ein Signalgenerator (80, 29, 63, 53) vorgesehen ist, der ein mindestens einem einstellbaren Betriebsparameter entsprechendes Signal erzeugt; und
f) der mindestens eine Signalgenerator (80, 29, 63, 53) über eine Datenübertragungsstrecke (84, 70, 76, 60, 50; 84; 106; 112) mit einem Dateneingang (93) der Dokumentationseinrichtung (88) verbunden ist.

2. Vorrichtung nach Anspruch 1, **dadurch gekennzeichnet, dass** ein Signalgenerator (80) an der Strahlungsquelle (12) vorgesehen ist, der ein wenigstens einem Betriebsparameter der Strahlungsquelle entsprechendes Signal erzeugt.

3. Vorrichtung nach Anspruch 1 oder 2, **dadurch gekennzeichnet, daß** ein steuerbares Netzteil (18) und/oder wenigstens ein steuerbarer Aktuator (28, 64) und/oder wenigstens eine weitere steuerbare Komponente (32) vorgesehen sind, deren Steuereingänge (72, 78, 62, 52) Daten über Datenübertragungsstrecken (70, 76, 60, 50) von der Dokumentationseinrichtung (88) erhalten.

4. Vorrichtung nach Anspruch 3, **dadurch gekennzeichnet, dass** der steuerbare Aktuator (28, 64) und/oder die weitere steuerbare Komponente (32) einen Signalgenerator (29, 63, 53) aufweisen, der ein mindestens einem jeweiligen Betriebparameter entsprechendes Signal erzeugt.

5. Vorrichtung nach einem der Ansprüche 1 bis 4, **dadurch gekennzeichnet, daß** wenigstens eine Datenübertragungsstrecke (84, 70, 76, 60, 50; 84; 106; 112) wenigstens in zwei Modi betreibbar ist, wobei in einem ersten Modus Ist-Betriebsparameter von dem wenigstens einen Signalgenerator (80, 29, 63, 53) zur Dokumentationseinrichtung (88) und/ oder einer Bildverarbeitungseinrichtung (40) übertragen werden und in einem zweiten Modus Soll-Betriebsparameter von der Dokumentationseinrichtung (88) und/oder der Bildverarbeitungseinrichtung (40) auf die Dateneingänge (72, 78, 62, 52) des steuerbaren Netzteils (18) und/oder des wenigstens einen steuerbaren Aktuators (28, 64) und/oder der wenigstens einen weiteren steuerbaren Komponente (32) gegeben werden.

6. Vorrichtung nach Anspruch 5, **dadurch gekennzeichnet, daß** das steuerbare Netzteil (18) und/oder wenigstens ein steuerbarer Aktuator (28, 64) und/oder wenigstens eine weitere steuerbare Komponente (32) und/oder die Dokumentationseinrichtung (88) und/oder die Bildverarbeitungseinrichtung (40) eine Kopie der von ihnen erhaltenen Daten wieder auf der Datenübertragungsstrecke (84, 70, 76, 60, 50; 84; 106; 112) bereitstellen.

7. Vorrichtung nach einem der Ansprüche 1 bis 6, **dadurch gekennzeichnet, daß** an wenigstens einem Ende einer Datenübertragungsstrecke (84, 70, 76, 60, 50, 44; 84, 94; 106; 112) eine digitale Schnittstelle (72, 68, 86, 82, 78, 74, 62, 58, 52, 48, 42, 46; 93, 96) vorgesehen ist.

8. Vorrichtung nach einem der Ansprüche 1 bis 7, **dadurch gekennzeichnet, daß** die Datenübertragungsstrecke (106; 112) eine galvanische Trennung gewährleistet.

9. Vorrichtung nach Anspruch 8, **dadurch gekennzeichnet, daß** die Datenübertragungsstrecke (106) zumindest in einem Teil eine optische Übertragungsstrecke (106) ist.

10. Vorrichtung nach Anspruch 9, **dadurch gekennzeichnet, daß** die Datenübertragungsstrecke (106) optoelektronische Bauelemente (102, 104) umfaßt.

11. Vorrichtung nach einem der Ansprüche 1 bis 8, **dadurch gekennzeichnet, daß** die Datenübertragungsstrecke (112) zumindest teilweise eine Funkstrecke (112) ist.

12. Vorrichtung nach einem der Ansprüche 1 bis 11, **dadurch gekennzeichnet, daß** der einstellbare Betriebsparameter die Röhrenspannung und/oder die Belichtungszeit und/oder den Strahlstrom und/oder die Gerätenummer und/oder die Wellenlänge der Strahlung und/oder den Strahlquerschnitt und/oder die Lage des Patienten oder dergleichen umfaßt.

13. Vorrichtung nach einem der Ansprüche 1 bis 12, **dadurch gekennzeichnet, daß** die Strahlungsquelle (12) Röntgenstrahlung bereitstellt.

14. Vorrichtung nach Anspruch 13, **dadurch gekennzeichnet, daß** der Bildwandler (32) wenigstens einen CCD-Sensor (34) umfaßt.

15. Vorrichtung nach Anspruch 13 oder 14, **dadurch gekennzeichnet, daß** der Bildwandler wenigstens eine Speicherfolie (34) umfaßt.

16. Vorrichtung nach einem der Ansprüche 1 bis 15, **dadurch gekennzeichnet, daß** ein steuerbares Netzteil (18) der Strahlungsquelle (12) bei einer Abweichung zwischen wenigstens einem Soll- und Ist-Betriebsparameter von der Dokumentationseinrichtung (88) und/oder der Bildverarbeitungseinrichtung (40) ein Signal erhält, welches den Betrieb der Strahlungsquelle (12) unterbindet.

17. Vorrichtung nach einem der Ansprüche 1 bis 16, **dadurch gekennzeichnet, daß** die räumliche Lage des Objektes (30) durch einen steuerbaren Aktuator (64) einstellbar ist.
